(19) Europäisches Patentamt
European Patent Office
Office européen des brevets

(11) **EP 2 529 757 B1**

(12) **EUROPEAN PATENT SPECIFICATION**

(45) Date of publication and mention
of the grant of the patent:
**08.01.2014 Bulletin 2014/02**

(51) Int Cl.:
***A61K 47/34*** *(2006.01)*   ***A61K 31/519*** *(2006.01)*
***A61K 9/00*** *(2006.01)*

(21) Application number: **12170366.4**

(22) Date of filing: **31.05.2012**

(54) **Paliperidone implant formulation**

Paliperidone Implantat Formulierung

Formulation d'implant de Paliperidone

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.05.2011 PCT/EP2011/059000
31.05.2011 PCT/EP2011/059001**

(43) Date of publication of application:
**05.12.2012 Bulletin 2012/49**

(73) Proprietor: **LABORATORIOS FARMACEUTICOS
ROVI, S.A.
28037 Madrid (ES)**

(72) Inventors:
• **Gutierro Aduriz, Ibon
28037 Madrid (ES)**
• **Franco Rodriguez, Guillermo
28037 Madrid (ES)**

(74) Representative: **Elzaburu Marquez, Alberto et al
Elzaburu, S.L.P.
C/ Miguel Ángel, 21
28010 Madrid (ES)**

(56) References cited:
**WO-A1-2011/151355     WO-A2-2008/153611
US-A1- 2005 042 294**

## Description

## TECHNICAL FIELD

The present invention relates to pharmaceutical composition for intramuscular injection comprising the drug paliperidone and/or its salts wherein the composition releases the drug with an immediate onset of action and continuously for at least 8 weeks, and wherein the composition has a pharmacokinetic profile in vivo that makes it suitable to be administered each 8 weeks or even longer periods. Specifically, the present invention is related to compositions for injectable *in-situ* forming biodegradable implants comprising paliperidone.

## BACKGROUND ART

Paliperidone is anatypical antipsychotic drug with benzisoxazole and piperidine functional groups, which act as strong dopaminergic antagonist and selective serotonin receptor antagonist. One of the intrinsic problems that paliperidone-targeted patients usually face is the dissociation of some schizophrenic patients from the treatment, moreover when it consists of a daily medication, leading to irregular or inconstant treatments and favouring the appearance of psychotic crisis. Moreover, this kind of therapy gives rise to high differences in the plasma levels (measured as the difference between Cmax and Cmin) in patients, therefore usually affecting the patient's mood.

Therefore, paliperidone is a good drug candidate for incorporation into sustained delivery devices, where the patients would be covered or treated for long time periods with just one dose and without the need of caregivers to pay attention to a daily medication, and where more homogeneous plasma levels in the patient are desirable. Other indications may involve bipolar mania and schizoaffective disorder, and its possible use in autism and Asperger's syndrome and Tourette's disorder may be of benefit to the patients.

Many patients with these mental illnesses achieve symptom stability with available oral antipsychotic medications; however, it is estimated that up to 75% have difficulty adhering to a daily oral treatment regimen, i.e. compliance problems. Problems with adherence often result in worsening of symptoms, suboptimal treatment response, frequent relapses and re-hospitalizations, and an inability to benefit from rehabilitative and psychosocial therapies.

Paliperidone recently received marketing approval as the first oral atypical antipsychotic with an extended release, which is achieved by an osmotic-controlled release oral delivery system. Paliperidone ER (WO2006/17537) is marketed as InvegaSustenna® and unsaturated derivatives thereof are described in WO2008/128436. Other extended release oral dosage forms for paliperidone are under development.

Due the presence of a secondary hydroxyl group, paliperidone may be provided as a prodrug. WO2009/15828 details acid-labile low molecular weight prodrugs of paliperidone intended to undergo hydrolysis in the stomach.

Therefore, in view of the state of the art, it is of interest to develop very long-acting, injectable depots of paliperidone. There is great need to improve the compliance factor particularly in the treatment of schizophrenia. The development of once-weekly or even longer acting injectable depot formulations of those drugs will mark a significant step forward to ensure continuous and steady supply of the effective medication.

EP2234617 describes ester-linked prodrugs of paliperidone to provide sustained plasma concentrations of paliperidone when administered once monthly, which may greatly enhance compliance with dosing. The substance paliperidone palmitate is approved as a once-monthly atypical antipsychotic intramuscular injection for treating schizophrenia and preventing recurrence of its symptoms. Paliperidone palmitate is formulated in a submicrocrystalline form. Paliperidone palmitate due to its dissolution rate-limited absorption exhibits flip-flop kinetics, where the apparent half-life is controlled by the absorption rate constant. Additionally the volume of injected drug product also impacts the apparent rate constant. It was also discovered that deltoid injections result in a faster rise in initial plasma concentration, facilitating a rapid attainment of potential therapeutic concentrations. Consequently, to facilitate patients' attaining a rapid therapeutic concentration of paliperidone it is preferred to provide the initial loading dose of paliperidone palmitate in the deltoids. The loading dose should be from about 100 mg-eq. to about 150 mg-eq. of paliperidone provided in the form of paliperidone palmitate. After the first or more preferably after the second loading dose injection patients will be approaching a steady state concentration of paliperidone in their plasma and may be injected in either the deltoid or the gluteal muscle thereafter. However, it is preferred that the patients receive further injections in the gluteal muscle. US2009/163519 outlines corresponding dosing regimen for long-acting injectable paliperidone esters of the palmitate type.

Other depot formulation it is described by the international application WO2011/42453. This specification describes a pharmaceutical composition for subcutaneous injection comprising a paliperidone compound. In particular the composition releases the paliperidone with an immediate onset of action and has an extended release time. Moreover, this specification relates to a pharmaceutical composition for subcutaneous injection comprising a paliperidone compound in a certain concentration.

WO 2008/153611 A2 discloses sustained delivery formulations for treating certain psychiatric conditions comprising risperidone. Finally, another antipsychotic injectable depot composition is described in the international application number WO2011/151355. This application is directed to a composition that can be used to deliver an antipsychotic prodrug of risperidone, such as paliperidone, as an injectable in-situ forming biodegradable implant for extended release providing therapeutic plasma levels from the first day. The composition is in the form of drug suspension on a biodegradable and biocompatible copolymer or copolymers solution using water miscible solvents that is administered in liquid form. Once the composition contacts the body fluids, the polymer matrix hardens retaining the drug, forming a solid or semisolid implant that releases the drug in a continuous manner.

Paliperidone was formulated as an implant as is described in WO2011/151355. However, after the data was analyzed from the clinical trials of this formulation it was discovered that the absorption of paliperidone from these injections was far more complex than was originally anticipated for obtaining a release the drug with an immediate onset of action and continuously for at least 8 weeks. Additionally, attaining a potential therapeutic plasma level of paliperidone in patients was discovered to be dependent on the molecular weight of the biocompatible polymer based on lactic and glycolic acid having a monomer ratio of lactic to glycolic acid. Due to the challenging nature of ensuring an optimum plasma concentration-time profile for treating patients with paliperidone, it is desirable to develop a dosing regimen that fulfills this goal in patients in need of treatment.

## SUMMARY OF THE INVENTION

Therefore, the compositions already described in the state of the art do not cover the existing needs in paliperidone compositions, kits and treatments for psychiatric disorders, and there still exists a need of compositions and devices to allow a controlled, constant release of the drug during prolonged periods of time during at least 8 weeks without a concomitant treatment or initial doses of risperidone and/or paliperidone.

The solution of this necessity is provided with an injectable intramuscular depot composition suitable for forming an in situ solid implant in a body, comprising a drug which is paliperidone, a biocompatible copolymer based on lactic and glycolic acid having a monomer ratio of lactic to glycolic acid in the range of 50:50 and a DMSO solvent, wherein the composition releases the drug with an immediate onset of action and continuously for at least 8 weeks and wherein the composition has a pharmacokinetic profile in vivo with substantially no burst release of the drug characterised in that the biocompatible copolymer has a molecular weight between 31 and 40 kDa and has an inherent viscosity in the range of 0.27-0.31dl/g.

In one aspect of the present invention there is provided a dosing regimen for administering paliperidone to a psychiatric patient in need of treatment comprising administering intramuscularly a first dose from about 75 mg to about 250 mg of paliperidone formulated in a sustained release formulation on the first day of treatment; administering intramuscularly other following doses from about 75 mg to about 250 mg of paliperidone formulated in a sustained release formulation between about the 56th to 65th day of treatment.

## DETAILED DESCRIPTION OF THE INVENTION

The compositions of the invention comprise at least a polymer or polymer matrix, a solvent and a drug.

One important aspect of this invention is an injectable intramuscular depot composition suitable for forming an in situ solid implant in a body, comprising a drug which is paliperidone or any pharmaceutically acceptable salt thereof in any combination, a biocompatible copolymer based on lactic and glycolic acid having a monomer ratio of lactic to glycolic acid in the range from 50:50 and a DMSO solvent, wherein the composition releases the drug with an immediate onset of action and continuously for at least 8 weeks and wherein the composition has a pharmacokinetic profile in vivo suitable to be administered between about 56th to 65th days after the preceding injection, characterised in that the biocompatible copolymer has a molecular weight between 31 and 40 kDa and has an inherent viscosity in the range of 0.27-0.31dl/g.

In a preferred embodiment of the invention, the biocompatible copolymer is gamma or beta irradiated in the dose range of 10-30 KGy measured at a temperature between -40ºC to +35ºC to adjust its molecular weight to a range between 31 and 40 kDa and its inherent viscosity to a range of of 0.27-0.31dl/g.

In a more preferred embodiment, the polymer is radiated at 15-25 KGy measured at the temperature of 8 ºC.

A preferrerd composition has the particle size distribution of the drug as follows:

- less than 10% particles smaller than 10 microns;
- less than 10% particles larger than 200microns, and
- a d0.5 value in the range of 40-90microns.

According to another embodiment of the invention, the drug/(polymer+drug) mass ratio is about 33% about 33%, the content of drug is about 13% w/w of total formulation, and the viscosity of solution between polymer and DMSO is in the range of 1.7 - 1.8 P.a.s.

In another embodiment, when the composition is formed, the drug is partially suspended with a solubility of drug in the DMSO solvent below 10 mg/ml.

In yet another embodiment, the composition is a sterile composition.

According to another aspect of the invention, the composition is a sterile composition and is suitable for the treatment of schizophrenia or bipolar disorders in the human body.

According to another aspect, the invention provides a pharmaceutical kit suitable for the in situ formation of a biodegradable implant in a body comprising the composition claimed, wherein the drug and the biocompatible polymer are contained in a first container, and the solvent is contained in a second, separate container. Preferably, at least one of the first and second containers is a syringe, a vial, a device or a cartridge, either disposable or not and more preferably both the first and the second containers are disposable syringes.

According to another aspect, the invention provides a method for the manufacturing of the said composition, comprising the step of providing a biocompatible copolymer having an initial polymer weight higher than required for the intramuscular depot composition and then adjusting its molecular weight to between 31 and 40kDa and its inherent viscosity to a range of 0.27-0.31 dl/g by irradiating it with gamma or beta radiation in the dose range of 10-30 KGy measured at a temperature between -40ºC and +35ºC.

Preferably, when the biocompatible polymer has an initial molecular weight of about 50 kDa, it is irradiated with a radiation dose of about 16KGy measured at 8ºC to reduce its molecular weight to between 35 and 38 kDa.

Preferably, when the biocompatible polymer has an initial molecular weight of about 63 kDa, it is irradiated with a radiation dose of about 30 KGy measured at 8ºC to reduce its molecular weight to between 30 and 36 kDa.

According to another aspect, the invention provides a dosing regimen method for administering an injectable intramuscular depot composition according to the invention to a patient in need of psychiatric treatment comprising:

a) administering intramuscularly to the patient a first dose in the amount of 75 mg to 250 mg of the injectable depot composition, at a point of time between the 24th day and the 35th day counting from the previous administration day;
b) administering a subsequent dose of the injectable depot composition in the amount of 75 mg to 250 mg from about the 56th day to about the 65th day after the administration of said first dose; and
c) repeating step b) whenever required.

Preferably, said first dose is about 100 mg to about 200 mg and this is equivalent than other doses.

Preferably, the steps a) and b) are repeated as many times as required.

The **polymer** or polymer matrix is preferably a biocompatible and biodegradable polymer matrix. In order not to cause any severe damage to the body following administration, the preferred polymers are biocompatible, non-toxic for the human body, not carcinogenic, and do not induce significant tissue inflammation. The polymers are preferably biodegradable in order to allow natural degradation by body processes, so that they are readily disposable and do not accumulate in the body. The preferred polymeric matrices in the practice in this invention are selected from end-capped terminal carboxylic poly-lactide and poly-glycolic acid copolymers mixed in a ratio of 50:50, with an average molecular weight in the range of 31 to 40 KDa and an inherent viscosity preferably in the range of 0.27-0.31dl/g.

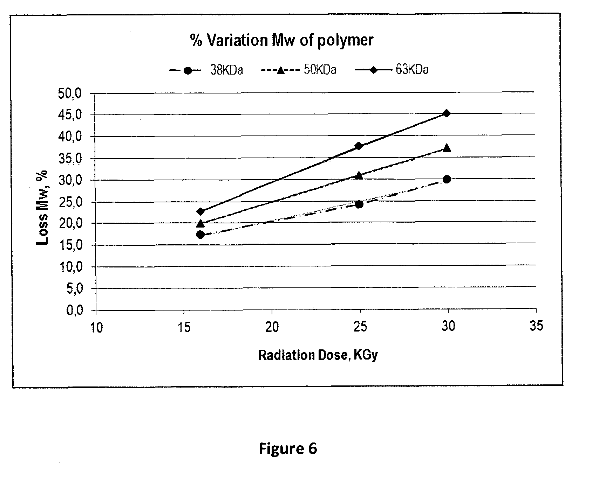

A commercial polymer with the required molecular weight can certainly be used. However, we have determined that the essential range of its molecular weight is between 31 and 40 kDa. Additionally we have determined in an in-house custom design that the molecular weight of the polymer can be varied by irradiating it with a radiation dose of between 10 and 30 kGy at a temperature between -40ºC and +35ºC, and this was not obvious in view of the state of the art to the skilled person (see figure 6). For example, the molecular weight of a commercially available polymer in a certain moment can be 50 KDa as an average value. We have determined a method for varying this molecular weight by irradiating the polymer with a certain dose of radiation that can be previously calculated. If done under controlled conditions, it is possible to obtain a mathematical model showing that the molecular weight of the polymer can be decreased with increasing irradiation doses.

Therefore, for example:

- When we need to use a PLGA polymer having a molecular weight between 31 and 40 kDa and an inherent viscosity value in the range of 0.27-0.31 dl/g, and we have as the starting polymer a polymer with 50 KDa of average molecular weight, we have determined that a radiation dose of 16 KGy is required to reduce its molecular weight to the cited range of 31-40 kDa, more preferably between 35-38 KDa.

- When we need to use a PLGA polymer having a molecular weight between 31 and 40 kDa and an inherent viscosity value in the range of 0.27-0.31 dl/g and we have as the starting polymer a polymer with 38 KDa of average molecular weight, we have determined that there is no need to use any radiation dose.

- When we need to use a PLGA polymer having a molecular weight between 31 and 40 kDa and an inherent viscosity value in the range of 0.27-0.31 dl/g, and we have as the starting polymer a polymer with 63 KDa of average molecular weight, we have determined that a radiation dose of 30 KGy is required to reduce its molecular weight to the cited range of 31 and 40 kDa, more preferably in the range of 31-36 kDa.

In these experimental tests, the temperature conditions for the polymer during the irradiation were about 8ºC. However, other temperatures can be used, such as e.g. lower than 35ºC, or lower than 25ºC, although in these cases the relationships between the radiation dose and the resulting molecular weight may vary.

The procedure is especially suitable for the manufacturing of the compositions described in the present invention. Furthermore, the filling of the solid polymer into syringes represents a real challenge in the manufacturing of injectable formulations. The polymer, manufactured as a non-sterile product, requires undergoing sterilization in order to achieve a formulation that can be injected into human beings. Probably the best way to solve this technical issue is to subject the polymer to sterilization by gamma or beta irradiation. Irradiation represents a challenging issue when using biodegradable polymers, as irradiation can disrupt the chains into fractions of smaller size. Control of the polymer molecular weight appears as again as the critical parameter to control the final characteristics of a product after a sterilization process.

As previously explained, chain size reduction by irradiation can be mathematically modelled or controlled in order to predict the final molecular weight of a polymer to be used as raw material having a molecular weight higher than desired. Therefore, once determined the fill weight of the polymer to be filled in a container (for example, the fill weight of the polymer in a syringe) and the bio-burden present in the polymer as raw material, the irradiation dose required to get the polymer sterile (as specified by ISO 11137) is selected for the required fill weight. Then the mathematical model describing the loss of molecular weight for a certain polymer versus the irradiated dose can identify the initial molecular weight of the polymer to be used as raw material required to obtain, after the irradiation process, a polymer with the desired final molecular weight for the formulation. As the availability of a polymer with a specific molecular weight can be somewhat limited, then we can alternatively select an available polymer with a molecular weight that is higher to what is required according to the irradiation dose identified, and then adjust the irradiation dose to a higher value in order to obtain a sterile polymer with the required molecular weight.

The concentration of the polymeric component in the compositions of the invention is preferably comprised in the range of 24-50%, (expressed as the percentage of polymer weight based on total polymeric solution component) and more preferably 25-27%.

For the purpose of the present invention, throughout the present specification the term intrinsic or inherent viscosity ($\eta_{inh}$) of the polymer is defined as the ratio of the natural logarithm of the relative viscosity, $\eta_r$, to the mass concentration of the polymer, c, i.e.:

$$\eta_{inh} = (\ln\eta_r)/c$$

and the relative viscosity ($\eta_r$) is the ratio of the viscosity of the solution $\eta$ to the viscosity of the solvent $\eta_s$, i.e.:

$$\eta_r = \eta / \eta_s$$

If not otherwise specified, the intrinsic viscosity and molecular weight values throughout the present specification are to be understood as measured with the method explained in example 1. The value of intrinsic viscosity is considered in the present specification, as commonly accepted in the art, as an indirect indicator of the polymer molecular weight. In this way, a reduction in the intrinsic viscosity of a polymer, measured at a given concentration in a certain solvent, with same monomer composition and terminal end groups, is an indication of a reduction in the polymer molecular weight (IUPAC. Basic definitions of terms relating to polymers 1974. Pure Appl. Chem. 40, 477-491 (1974).

The preferred **solvents** are non-toxic, biocompatible and appropriate for parenteral injection. Solvents susceptible of causing toxicity should not be used for the injection of any material into any living body. More preferably, selected solvents are biocompatible in order not to cause any severe tissue irritation or necrosis at the injection site. Therefore, the solvent is preferably classified as class II or III, and more preferably class III, according to ICH Guidelines. For the formation of the *in-situ* implant, the solvent should preferably diffuse quickly from the polymeric solution towards surrounding tissues when is exposed to physiological fluids. Consequently, the solvent is preferably DMSO.

The **drug** is preferably paliperidone and/or its pharmaceutically acceptable salts and combinations thereof. This drug is preferably partially suspended in the solvent. Partially suspended means that the solubility of drug in the DMSO solvent is preferably below 10 mg/ml, when the formulation or implant is formed, is below 10 mg/ml in the total volume of DMSO and calculated at 25 ºC. The advantage of this low solubility is that the initial burst of the drug when the solvent diffuses to the external aqueous medium is greatly reduced. In addition, in the final compositions of the invention the drug is provided in a preferred concentration between 4 and 16 wt%, expressed as the percentage of the drug in respect of the total composition weight. More preferably, the drug content is between 7 and 15%, and most preferably about 13% in respect of the total composition weight.

One of the factors contributing to control the initial release of the composition of the invention is the viscosity of the polymeric solution. The "polymeric solution", which is defined as the combination of the polymer matrix and the solvent where it is dissolved, has a preferred viscosity in the range of 1.6 - 2.5Pa.s, more preferably between 1.7-2.1Pa.s.

A second factor contributing to control the initial release of the compositions of the invention is the biocompatible copolymer molecular weight that must be between 31 and 40 kDa . The adequate balance in this composition between drug solubility in the solvent and the molecular weight of the polymer in the implant (that controls the polymer precipitation process and the final structural characteristics of the implant) allows the formulation to limit the amount of paliperidone that can be released in the solvent diffusion phase after the intramuscular injection. Once the formulation is injected in the intramuscular tissue, the DMSO is rapidly dissolved in the surrounding aqueous environment. The relative increase of the polymer concentration in DMSO over the polymer solubility in the solvent leads to the formation of a polymer precipitate that entraps the paliperidone that was not solubilized in the solvent. Molecular weight of the polymer has a great impact in this critical step, as too low weighed chains have delayed precipitation time compared to the chains having the weight in the adequate range. This delayed precipitation allows the drug to increase contact with the surrounding fluids towards the drug is being released. Therefore, low molecular weighed chains lead to an excessive release of paliperidone after the injection and potentially to obtain toxic plasma levels on the first days after the injection and/or to obtain a formulation that is not capable to cover a minimum of 8 weeks between injections. Molecular weight of the polymer also can affect the release of the drug from the intramuscularly injected implant after solvent diffusion and polymer precipitation. Molecular weights over the specified range are not capable to maintain adequate release rates of paliperidone by diffusion. Additionally, higher molecular weight chains in the intramuscular tissue require longer hydrolysis times in order to provide soluble fractions that could release the drug entrapped in the polymer matrix. A higher remaining drug content to be released could lead to the obtention of undesirably high active moiety plasma values, or plasma values after 60 days post injection that could somehow interfere with the following dose as the formulation is intended to be injected several times into the human, each 8 weeks or more days.

One important aspect of this invention is an injectable intramuscular depot composition suitable for forming an in situ solid implant in a body, comprising a drug which is paliperidone or any pharmaceutically acceptable salt thereof in any combination, a biocompatible copolymer based on lactic and glycolic acid having a monomer ratio of lactic to glycolic acid in the range of 50:50 and a DMSO solvent, wherein the composition releases the drug with an immediate onset of action and continuously for at least 8 weeks and wherein the composition has a pharmacokinetic profile in vivo suitable to be administered between about 56th to 65th days after the preceding injection, characterised in that the biocompatible copolymer has a molecular weight to a range between 31 and 40 kDa and has an inherent viscosity to a range of of 0.27-0.31dl/g.

In the compositions cited previously, the polymer is radiated at a temperature lower than 35 º C, more preferably lower than 25 and more preferably lower than 8ºC

This composition has the preferred particle size distribution of the drug as follows:

- less than 10% particles smaller than 10 microns;
- less than 10% particles larger than 200microns, and
- a d0.5 value in the range of 40-90microns.

According to an embodiment, the drug / (polymer+drug) mass ratio is about 33% about 33%, the content of drug is about 13% w/w of total formulation, and the viscosity of solution between polymer and DMSO is in the range of 1.7 - 1.8 P.a.s.

According to another embodiment, when the composition is formed, the drug is partially suspended with a solubility of drug in the DMSO solvent below 10 mg/ml.

According to yet another embodiment, the composition is a sterile composition and is suitable for the treatment of schizophrenia or bipolar disorders in the human body.

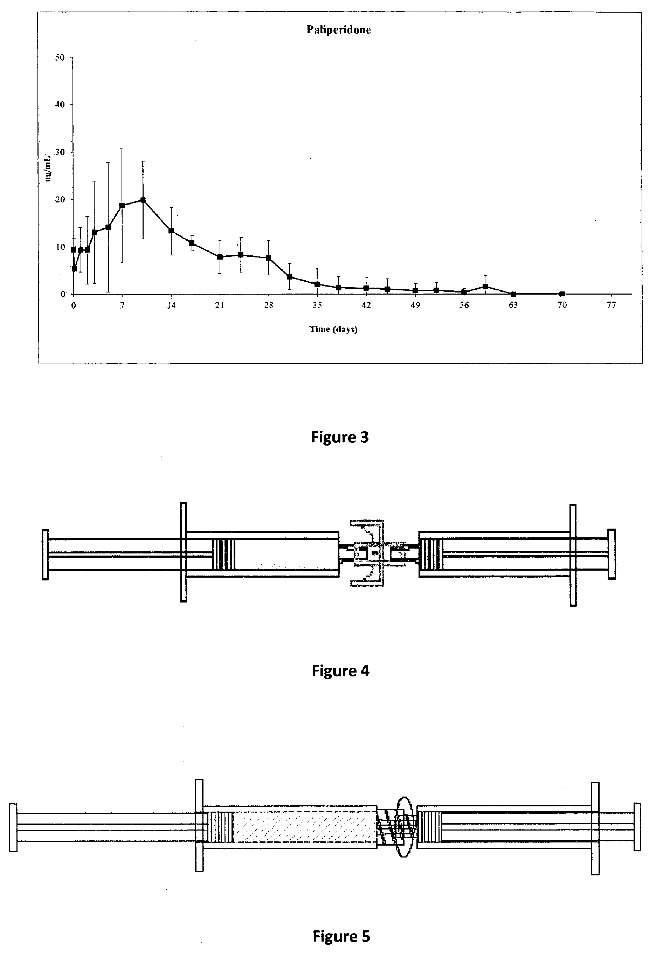

According to another aspect the invention provides a pharmaceutical kit suitable for the in situ formation of a biodegradable implant in a body comprising the composition claimed, wherein the drug and the biocompatible polymer are contained in a first container, and the solvent is contained in a second, separate container. Preferably, at least one of the first and second containers is a syringe, a vial, a device or a cartridge, either disposable or not and more preferably both the first and the second containers are disposable syringes. This aspect of the invention is directed to a kit comprising a first container, preferably syringes, vials, devices or cartridges, all of them either being disposable or not, containing a polymer in solid form, such as PLGA and a drug in the appropriate amounts and a second container, likewise preferably syringes, vials, devices or cartridges, all of them being either disposable or not, containing the water-miscible solvent. When required, the contents of both containers are combined, for example through a connector or by using male-female syringes, and mixed each other so that the compositions according to the invention are reconstituted, for example by moving forwards and backwards the plungers of the syringes. Illustrative preferred embodiments are shown in Figure 4 (syringes connected through a connector device) and in Figure 5 (syringes connected through a direct thread).

According to another aspect, the invention provides a method for the manufacturing of the composition claimed comprising the step of providing a biocompatible copolymer having a polymer weight higher than required for the intramuscular depot composition and then adjusting its molecular weight to between 31 and 40kDa and its inherent viscosity to a range of 0.27-0.31 dl/g by irradiating it with gamma or beta radiation in the dose range of 10-30 KGy measured at a temperature between -40ºC and +35ºC.

Preferably, when the biocompatible polymer has an initial molecular weight of about 50 kDa, it is irradiated with a radiation dose of about 16KGy to reduce its molecular weight to between 35 and 38 kDa.

Preferably, when the biocompatible polymer has an initial molecular weight of about 63 kDa, it is irradiated with a radiation dose of about 30 KGy to reduce its molecular weight to between 30 and 36 kDa.

According to another aspect the invention provides a dosing regimen method for administering an injectable intramuscular depot composition according to the invention to a patient in need of psychiatric treatment comprising:

a) administering intramuscularly to the patient a first dose in the amount of 75 mg to 250 mg of the injectable depot composition, at a point of time between the 24th day and the 35th day counting from the previous administration day;
b) administering a subsequent dose of the injectable depot composition in the amount of 75 mg to 250 mg from about the 56th day to about the 65th day after the administration of said first dose; and
c) repeating step b) whenever required.

Preferably, said first dose is about 100 mg to about 200 mg and this is equivalent than other doses.

In a preferred embodiment, the injectable depot composition is sterile as a finished product. In other preferred embodiment, the biocompatible polymer is sterilized previously to its aseptic filling process, preferably by irradation in the range 15-30 KGy.

**BRIEF DESCRIPTION OF THE FIGURES**

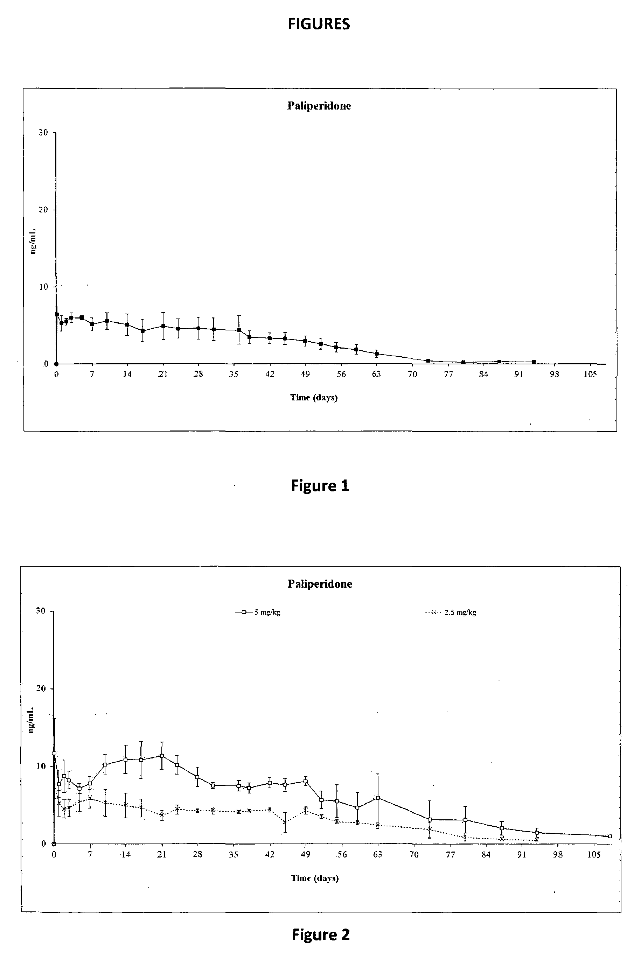

Fig 1.- Paliperidone levels profile in dog after the administration of the paliperidone formulation described in example 1 to Beagle dogs (n=3). Dose is 2.5 mg/kg. Results are expressed as ng/ml plasma values of paliperidone versus time. The table describes Area Under the Curve (AUC) of paliperidone plasma levels versus time. AUC all as well as AUC vs three different time frames are included. Units are expressed in h*ng/ml.

Fig 2.- Paliperidone levels profile in dog after the administration of the paliperidone formulation described in example 2 to two cohorts of Beagle dogs (each cohort n=6). Doses were 2.5 and 5 mg/kg. Results are expressed as ng/ml plasma values of paliperidone versus time. The table describes Area Under the Curve (AUC) of paliperidone plasma levels versus time. AUC all as well as AUC vs three different time frames are included. Units are expressed in h*ng/ml.

Fig 3.- Paliperidone levels profile in dog after the administration of the paliperidone formulation described in example 3 to Beagle dogs (n=3). Dose is 2.5 mg/kg. Results are expressed as ng/ml plasma values of paliperidone versus time. The table describes Area Under the Curve (AUC) of paliperidone plasma levels versus time. AUC all as well as AUC vs three different time frames are included. Units are expressed in h*ng/ml.

Fig 4.- Drawing of a kit suitable for the preparation of paliperidone compositions comprising two male syringes linked by a connector. Polymer+paliperidone are contained in one syringe and DMSO filled in the second syringe.

Fig 5.-Drawing of a kit suitable for the preparation of paliperidone compositions comprising a female syringe linked to a male syringe. Polymer+paliperidone can be contained in one syringe and DMSO filled in the second syringe. Preferably female syringe contains polymer+paliperidone as a solids and male syringe is filled with DMSO.

Fig 6.- Loss of molecular weight percentage in the custom design. The molecular weight of the polymer can be varied by irradiating it with a certain radiation dose. The table describes the percentage of loss of polymer weight versus radiation dose.

**EXAMPLES**

The following examples illustrate the invention and should not be considered in a limitative sense thereof.
In the sense of the present invention, without limitation and in connection with the *in vivo* examples, for "Initial Burst" or initial release it is meant the addition of the plasma levels of paliperidone, from the moment of the injection until the third day after the administration. In a similar way, an "adequate plasma level profile" is considered as not more than the 45% of the AUC of the paliperidone occurring between the moment of the injection until day 21, between 35% and 45% of the AUC of the paliperidone occurring between day 21 and day 49, and not more than 35% of the AUC of the paliperidone occurring after day 49. These percentages represent an adequate balance between the different periods in which paliperidone is being released from the implant in order to have a formulation to be injected each 8W or each 60 days capable to obtain therapeutic plasma levels of paliperidone in a human since the first day of the injection, capable to obtain the desired average paliperidone plasma concentrations during the period between injections and with reduced peak-valley plasma values of paliperidone that could lead to toxicity or lack of efficacy. Also in the sense of this invention, without limitation and in connection with the examples, acceptable plasma levels of paliperidone during the initial burst phase are below 75 ng/ml in Beagle dogs when doses administered are 2.5 mg/kg paliperidone.

**Example 1: Depot formulation with Resomer® 503**

In the present example, the following formulation was prepared:

| | Ingredient | Amount (mg) |
|---|---|---|
| Female 2.25 ml syringe | Lactic-co-glycolic acid copolymer (N-capped) with 50% content of each of the two organic acid monomers and a molecular weight of 32.5KDa | 50 |
| | Paliperidone | 25 |
| Male 2.25 ml syringe | Ingredient | Amount (mg) |
| | Dimethyl sulfoxide | 117 |

Paliperidone particle size was characterized by light scattering and provided the following distribution of particle size: d(0.1) = 17.41 $\mu$m, d(0.5) = 51.61 $\mu$m and d(0.9) = 175.32 $\mu$m.
Polymer has been characterized for its molecular weight according to the following technique:

Equipment

GPC chromatograph with triple detector (laser diffraction, viscosimetry, refraction index)

• Viscotek® GPCmax VE 2001 GPC SOLVENT/SAMPLE MODULE

• Viscotek® TDA 305 TRIPLE DETECTOR ARRAY

Reagents

• Tetrahydrofurane (THF) grade GPC stabilized with butyl hydroxyl toluene (BHT) 250 ppm
• 99 KDa polystyrene standard

Sample preparation

• 10 mg/ml Standard Sample

- 10 mg/ml Test sample: 3 samples for each polymer to be tested

Pre-conditioning

Condition and stabilize column and detectors with mobile phase (THF) until reaching working flow rate of 1 ml/min and purge viscometer and refraction index detectors, checking at the end that all signals are stable and adequate.

Chromatographic conditions:

- Column: i-MBMMW-3078 (CLM1012, Viscotek)

- Delay column: medium delay (CLM9002, Viscotek)

- Column temperature 30°C

- Flux rate 1 ml/min

- Injection volume: 100 $\mu$l

- Run time: 15 minutes

- Eluent: stabilized THF (pre-heated to 30°C and under 100 rpm agitation)

System verification

- Inject 100 $\mu$l of eluent and check there is no response in signals related with molecular weight determination

- Inject 100 $\mu$l of polystyrene standard and check adequacy of the measurement. Repeat at least twice.

Acceptance Criteria: $\pm$2% of the nominal Molecular Weight and Intrinsic Viscosity declared by manufacturer standard certificate.

Calibration

Not necessary if system verification complies and no previous chromatographic conditions are changed.
In case it would be required to calibrate:

- Inject 100 $\mu$l of polystyrene standard at least twice.

- Use first sample's data for triple calibration by creating a new multidetectors - homopolymer's method.

- Introduce into the method all the data needed for internal calibration such standard values of MW, IV, dn/dc, dA/dc and refractive index of the solvent.

- Calibrate the system as the equipment specify and save the new method.

- Check with the new method the adequacy of the measurement for the second injection of the standard.

Procedure

Inject by triplicate 100 $\mu$l of the test sample

Polymer molecular weight measured according to the technique specified resulted in 32.5KDa. According to a similar technique, inherent viscosity of the polymer resulted in a value of 0.27dl/g. It is important to mention that inherent viscosity values correspond to those obtained with the technique described, specially related to temperature conditions and eluent used. Any change in measurement conditions mean the obtention of different values as directly depend on them.

The paliperidone implantable formulation was prepared by connecting male and female syringes and moving the plungers forwards and backwards upon complete dissolution of the polymer and the formation of a homogeneous suspension of the paliperidone in the polymer dissolution.

*In vivo plasma levels after intramuscular administration to Beagle dog:*

The paliperidone composition of this example was intramuscularly injected to Beagle dogs weighing an average of 10 kg. The amount injected corresponded to a dose of 25 mg paliperidone and the composition was intramuscularly placed in the left hind leg using a syringe with a 20G needle. Total number of dogs was 3. After injection, plasma levels were obtained at 0, 4h, 1d, 2d, 3d, 5d, 7d, 10d, 14d, 17d, 21d, 24d, 28d, 31d, 36d, 38d, 42d, 45d, 49d, 52d, 55d, 59d, 63d, 73d, 80d, 87d, 94d, 108d and 118d.

The kinetics of the plasma levels corresponding to the paliperidone was evaluated by tandem mass HPLC. The profile of the plasma levels of the paliperidone is shown in Figure 1. The results are expressed as paliperidone concentrations (ng/ml) as the function of time. As it can be observed in this Figure, the injection of an amount of composition equivalent to 25 mg paliperidone to Beagle dogs resulted in very high control of the initial burst release followed by a slow, sustained decrease, with continuous plasma levels from day 1 onwards. The plasma levels profile for the paliperidone, as previously described, can be considered adequate as provide very low risk of having toxic plasma levels just after the injection. This property is crucial for a formulation intended to be administered at least each 8 weeks, as the dose needed to be injected is high compared to existing monthly regimens and, therefore, the risk of reaching toxic plasma levels with small variations of the drug fraction released at this stage is potentially very high. In this formulation, drug release during polymer precipitation process is controlled by the drug solubility in the solvent, the amount of solvent (ratio drug to solvent) and the polymer concentration and molecular weight.

The results also indicate that the formulation can release paliperidone over a period of more than two months in a prolonged manner, with reduced peak to valley plasma concentration fluctuations of the drug. An adequate control of the drug to polymer ratio and the polymer molecular weight are crucial to achieve this target.

Once the formulation is injected in the intramuscular tissue, the DMSO is rapidly dissolved in the surrounding aqueous environment. The relative increase of the polymer concentration in DMSO over the polymer solubility in the solvent leads to the formation of a polymer precipitate that entraps the paliperidone that was not solubilized in the solvent. Molecular weight of the polymer has a great impact in this critical step, as too low weighed chains have delayed precipitation time compared to the chains having the weight in the adequate range. This delayed precipitation allows the drug to increase contact with the surrounding fluids towards the drug is being released. Therefore, low molecular weighed chains lead to an excessive release of paliperidone after the injection and potentially to obtain toxic plasma levels on the first days after the injection. Molecular weight of the polymer also can affect the release of the drug from the intramuscularly injected implant after solvent diffusion and polymer precipitation. Molecular weights over the specified range are not capable to maintain adequate release rates of paliperidone by diffusion. This phase is particularly prolonged in a bi-monthly formulation compared to a formulation intended to be administered each 4 weeks, and the control of the polymer molecular weight becomes particularly crucial. Additionally, higher molecular weight chains can lead to formulations that are too viscous to be injected at the required concentration (more than 3.0 Pa.s) and once injected in the intramuscular tissue require longer hydrolysis times in order to provide soluble fractions that could release the drug entrapped in the polymer matrix. A higher remaining drug content to be released could lead to the obtention of undesirably low paliperidone plasma values, between days 24 and 49 that could lead to obtain under therapeutic plasma concentrations of the drug.

| | AUC all | AUC $_{0-21}$ days | AUC $_{21-49}$ days | AUC $_{42-last}$ |
|---|---|---|---|---|
| | (h*ng/ml) | (h*ng/ml) | (h*ng/ml) | (h*ng/ml) |
| **Dose 2.5 mg/kg** | 5985.5 | 2615.9 | 2666.28 | 703.32 |

**Example 2: Depot formulation with Resomer® 504 radiated to 16KGy. 2 doses.**

Preparation of sterile formulations to be used in vivo can require the use of procedures, such as irradiation, that have the potential to affect the polymer molecular structure. The example shows that the polymer molecular weight loss that occur upon polymer irradiation can be measured and controlled in order to achieve a sterile polymer with the desired characteristics in order to obtain adequate plasma levels profile of paliperidone. In this example, a lactic-co-glycolic acid copolymer with 50% content of each of the two organic acid monomers and a molecular weight of 50 KDa was sterilized by beta irradiation at 16 KGy under controlled temperature and moisture conditions. The resultant polymer was characterized for its molecular weight according to the method described in example 1. Molecular weight after irradiation process was 40KDa

| | Ingredient | Amount (mg) |
|---|---|---|
| Female 2.25 ml syringe | Lactic-co-glycolic acid copolymer (N-capped) with 50% content of each of the two organic acid monomers and a molecular weight of 50 KDa, beta-irradiated as a bulk with a 16 KGy dose achieving a final molecular weight of 40KDa. | 100 |
| | Paliperidone | 50 |
| Male 2.25 ml syringe | Ingredient | Amount (mg) |
| | Dimethyl sulfoxide | 234 |

Paliperidone particle size was characterized by light scattering and provided the following distribution of particle size: d(0.1) = 17.41 $\mu$m, d(0.5) = 51.61 $\mu$m and d(0.9) = 175.32 $\mu$m.

Inherent viscosity of the irradiated polymer, as calculated by the technique described in example 1 was 0.31dl/g.

The paliperidone implantable formulation was prepared by connecting male and female syringes and moving the plungers forwards and backwards upon complete dissolution of the polymer and the formation of a homogeneous suspension of the paliperidone in the polymer dissolution.

*In vivo plasma levels after intramuscular administration to Beagle dog:*

The paliperidone composition of this example was intramuscularly injected to two cohorts of Beagle dogs weighing an average of 10 kg. The amount injected corresponded to a dose of 25 mg paliperidone in one cohort and to a dose of 50 mg paliperidone in a second cohort. The compositions were intramuscularly placed in the left hind leg using a syringe with a 20G needle. Total number of dogs was 3 for each cohort. After injection, plasma levels were obtained at 0, 4h, 1d, 2d, 3d, 5d, 7d, 10d, 14d, 17d, 21d, 24d, 28d, 31d, 36d, 38d, 42d, 45d, 49d, 52d, 55d, 59d, 63d, 73d, 80d, 87d, 94d, 108d and 118d.

The kinetics of the plasma levels corresponding to the paliperidone was evaluated by tandem mass HPLC. The profile of the plasma levels of the paliperidone is shown in Figure 2. The results are expressed as paliperidone concentrations (ng/ml) as the function of time. As it can be observed in this Figure, the injection of an amount of composition equivalent to 25 mg paliperidone to Beagle dogs resulted in a paliperidone plasma levels profile that was similar to that observed in Example 1, representing a polymer that was not irradiated.

The example shows how can be adjusted by the sterilization process the polymer molecular weight in order to obtain a polymer that is sterile, and that confers the formulation the desired release properties. Possible changes in terminal end groups of the polymer after irradiation process do not significantly change in vivo release properties in the specified range, meaning that molecular weight is the major factor that controls degradation process in this composition.

| | AUC all (h*ng/ml) | AUC $_{0-21}$ days (h*ng/ml) | AUC $_{21-49}$ days (h*ng/ml) | AUC $_{42-last}$ (h*ng/ml) |
|---|---|---|---|---|
| Dose 5 mg/kg | 14991.22 | 4842.82 | 5521.8 | 4626.6 |
| Dose 2.5 mg/kg | 7077.38 | 2503.94 | 2708.16 | 1865.28 |

**Example 3: Depot formulation with Resomer® 504 radiated to 25KGy**

The current example demonstrates that an excessive reduction in the polymer molecular weight can lead to a shortening of the duration of paliperidone plasma levels profile that makes the formulation not suitable to be administered each 8 weeks or more.

A lactic-co-glycolic acid copolymer with 50% content of each of the two organic acid monomers and a molecular weight of 38KDa was sterilized by beta irradiation at 25KGy under controlled temperature and moisture conditions. The resultant polymer was characterized for its molecular weight according to the method described in example 1. Molecular weight after irradiation process was 29KDa.

| Female 2.25 ml syringe | Ingredient | Amount (mg) |
|---|---|---|
| | Lactic-co-glycolic acid copolymer (N-capped) with 50% content of each of the two organic acid monomers and a molecular weight of 38KDa, beta-irradiated as a bulk with a 25 KGy dose achieving a final molecular weight of 29KDa. | 50 |
| | Paliperidone | 25 |
| Male 2.25 ml syringe | Ingredient | Amount (mg) |
| | Dimethyl sulfoxide | 117 |

Paliperidone particle size was characterized by light scattering and provided the following distribution of particle size: d(0.1) = 17.41 $\mu$m, d(0.5) = 51.61 $\mu$m and d(0.9) = 175.32 $\mu$m.

Inherent viscosity of the irradiated polymer, as calculated by the technique described in example 1 was 0.31 dl/g.

The paliperidone implantable formulation was prepared by connecting male and female syringes and moving the plungers forwards and backwards upon complete dissolution of the polymer and the formation of a homogeneous suspension of the paliperidone in the polymer dissolution.

*In vivo plasma levels after intramuscular administration to Beagle dog:*

The paliperidone composition of this example was intramuscularly injected to Beagle dogs weighing an average of 10 kg. An amount of formulation equivalent to a dose of 2.5 mg/kg of paliperidone was intramuscularly placed in the left hind leg using a syringe with a 20G needle. Total number of dogs per cohort was 3. After injection, plasma levels were obtained at 0, 4h, 1d, 2d, 3d, 5d, 7d, 10d, 14d, 17d, 21d, 24d, 28d, 31d, 35d, 38d, 42d, 45d, 49d,52d,56d,59d,63d,70d,77d.

The kinetic of the plasma levels corresponding to paliperidone was evaluated and is shown in Figure 3. The results are expressed as paliperidone concentrations (ng/ml) as the function of time. As it can be observed in this Figure, the injection of an amount of composition equivalent to 2.5 mg/kg to Beagle dogs resulted in a shortened plasma levels profile. A reduction of 4 KDa from 33to 29increases significantly drug diffusion throughout the polymer matrix, leading to a reduced availability of the drug to be released after 30 days post injection.

| | AUC all | AUC $_{0-21}$days | AUC $_{21-49}$days | AUC $_{42-last}$ |
|---|---|---|---|---|
| | (h*ng/ml) | (h*ng/ml) | (h*ng/ml) | (h*ng/ml) |
| Dose 2.5 mg/kg | 10542.26 | 7142.06 | 3128.04 | 272.16 |

## Claims

1. An injectable intramuscular depot composition suitable for forming an in situ solid implant in a body, comprising a drug which is paliperidone and/or its pharmaceutical acceptable salts in any combination thereof, a biocompatible copolymer based on lactic and glycolic acid having a monomer ratio of lactic to glycolic acid in the range of 50:50 and DMSO as solvent, wherein the composition releases the drug with an immediate onset of action and continuously for at least 8 weeks and wherein the composition has a pharmacokinetic profile *in vivo* suitable for the formulation to be administered each 8 weeks or longer periods, **characterised in that** the biocompatible copolymer has a molecular weight in the range of between 31 and 40 kDa and an inherent viscosity in the range of between 0.27-0.31 dl/g.

2. The composition of claim 1, wherein the biocompatible copolymer is gamma or beta irradiated in the dose range of 10-30 KGy measured at a temperature between -40ºC and +35ºC to adjust its molecular weight to a range between 31 and 40 kDa and its inherent viscosity to a range of between 0.27-0.31 dl/g.

3. The composition claims 1 or 2, wherein the particle size distribution of the drug is:

   - less than 10% particles smaller than 10 microns;
   - less than 10% particles larger than 225 microns, and
   - a d0.5 value in the range of 40-130 microns.

4. The composition according to any one of claims 1 to 3, wherein the drug/(polymer+drug) mass ratio is about 33%.

5. The composition according to any one of claims 1 to 4, wherein the content of drug is about 13% w/w of total formulation and the viscosity of the solution comprising the polymer and the DMSO is in the range of 1.7 - 1.8 P.a.s.

6. The composition of any one of previous claims for the treatment of schizophrenia or bipolar disorders in the human body.

7. A pharmaceutical kit suitable for the in situ formation of a biodegradable implant in a body comprising the composition of any one of claims 1-6, wherein the drug and the biocompatible polymer are contained in a first container, and the solvent is contained in a second, separate container.

8. The pharmaceutical kit according to claim 7, wherein at least one of the first and second containers is a syringe, a vial, a device or a cartridge, either disposable or not.

9. A method for the manufacturing of a composition according to any one of previous claims 1-6, comprising the step of providing a biocompatible copolymer having an initial polymer weight higher than required for the intramuscular depot composition and then adjusting its molecular weight to between 31 and 40kDa and its inherent viscosity to a range of 0.27-0.31 dl/g by irradiating it with gamma or beta radiation in the dose range of 10-30 KGy measured at a temperature between -40ºC and +35ºC.

10. Method according to claim 9 wherein the dose range irradiated to the polymer is in the range of 16-25 KGy measured at the temperature of 8ºC.

11. Method according to claims 9 or 10 wherein, when the biocompatible polymer has an initial molecular weight of about 50 kDa, it is irradiated with a radiation dose of about 16 KGy to reduce its molecular weight to between 35 and 38 kDa.

12. Method according to claims 9 or 10 wherein, when the biocompatible polymer has an initial molecular weight of about 63 kDa, it is irradiated with a radiation dose of about 30 KGy to reduce its molecular weight to between 30 and 36 kDa.

**Patentansprüche**

1. Als intramuskuläres Depot injizierbare Zusammensetzung, die zur Bildung eines festen In-situ-Implantats in seinem Körper geeignet ist und die einen Wirkstoff, bei dem es sich um Paliperidon und/oder um eines seiner pharmazeutisch verträglichen Salze in einer beliebigen Kombination davon handelt, ein biokompatibles Copolymer auf Basis von Milchsäure und Glykolsäure mit einem Monomerverhältnis von Milchsäure zu Glykolsäure im Bereich von 50:50 und DMSO als Lösungsmittel umfasst, wobei die Zusammensetzung den Wirkstoff mit einer sofortigen Wirkung und kontinuierlich für mindestens acht Wochen freisetzt und wobei die Zusammensetzung *in vivo* ein pharmakokinetisches Profil hat, das zum Verabreichen der Formulierung alle acht Wochen oder über längere Zeiträume geeignet ist, **dadurch gekennzeichnet, dass** das biokompatible Copolymer ein Molekulargewicht im Bereich von 31 bis 40 kDa und eine inhärente Viskosität im Bereich von 0,27 bis 0,31 dl/g aufweist.

2. Zusammensetzung nach Anspruch 1, wobei das biokompatible Copolymer gamma- oder beta-bestrahlt ist, im Dosisbereich von 10 bis 30 kGy, gemessen bei einer Temperatur von -40 °C bis +35 °C, um sein Molekulargewicht auf einen Bereich von 31 bis 40 kDa und seine inhärente Viskosität auf einen Bereich von 0,27 bis 0,31 dl/g einzustellen.

**3.** Zusammensetzung nach Anspruch 1 oder 2, wobei die Teilchengrößenverteilung des Wirkstoffs wie folgt ist:

- weniger als 10 % der Teilchen sind kleiner als 10 Mikron;
- weniger als 10 % der Teilchen sind größer als 225 Mikron und
- ein d0,5-Wert liegt im Bereich von 40 bis 130 Mikron.

**4.** Zusammensetzung nach einem der Ansprüche 1 bis 3, wobei das Massenverhältnis von Wirkstoff zu (Polymer + Wirkstoff) bei etwa 33 % liegt.

**5.** Zusammensetzung nach einem der Ansprüche 1 bis 4, wobei der Wirkstoffgehalt etwa 13 Gew.-% der gesamten Formulierung ausmacht und die Viskosität der Lösung, die das Polymer und das DMSO umfasst, im Bereich von 1,7 bis 1,8 Pa s liegt.

**6.** Zusammensetzung nach einem der vorhergehenden Ansprüche zur Behandlung von Schizophrenie oder manisch-depressiven Psychosen im menschlichen Körper.

**7.** Pharmazeutisches Kit, das zur In-situ-Bildung eines biologisch abbaubaren Implantats in einem Körper geeignet ist und das die Zusammensetzung nach einem der Ansprüche 1 bis 6 umfasst, wobei der Wirkstoff und das biokompatible Polymer in einem ersten Behälter enthalten sind und das Lösungsmittel in einem zweiten, separaten Behälter enthalten ist.

**8.** Pharmazeutisches Kit nach Anspruch 7, wobei der erste und/oder der zweite Behälter eine Spritze, eine Ampulle, eine Vorrichtung oder eine Kartusche ist, entweder ein Einwegprodukt oder nicht.

**9.** Verfahren zur Herstellung einer Zusammensetzung nach einem der vorhergehenden Ansprüche 1 bis 6, das den Schritt des Bereitstellens eines biokompatiblen Copolymers mit einem anfänglichen Polymergewicht, das höher als für die Zusammensetzung als intramuskuläres Depot erforderlich ist, und dann des Einstellens seines Molekulargewichts auf einen Wert von 31 bis 40 kDa und seine inhärente Viskosität auf einen Bereich von 0,27 bis 0,31 dl/g durch Bestrahlen dieses mit Gamma- oder Betastrahlung im Dosisbereich von 10 bis 30 kGy, gemessen bei einer Temperatur von -40 °C bis +35 °C, umfasst.

**10.** Verfahren nach Anspruch 9, wobei der Dosisbereich, mit dem das Polymer bestrahlt wird, im Bereich von 16 bis 25 kGy, gemessen bei der Temperatur von 8 °C, liegt.

**11.** Verfahren nach Anspruch 9 oder 10, wobei, wenn das biokompatible Polymer ein anfängliches Molekulargewicht von etwa 50 kDa aufweist, es mit einer Strahlungsdosis von etwa 16 kGy bestrahlt wird, um sein Molekulargewicht auf einen Wert von 35 bis 38 kDa zu senken.

**12.** Verfahren nach Anspruch 9 oder 10, wobei, wenn das biokompatible Polymer ein anfängliches Molekulargewicht von etwa 63 kDa aufweist, es mit einer Strahlungsdosis von etwa 30 kGy bestrahlt wird, um sein Molekulargewicht auf einen Wert von 30 bis 36 kDa zu senken.

## Revendications

**1.** Une composition à effet retard pour injection intramusculaire adaptée pour former un implant solide *in situ* dans un corps, comprenant un médicament qui est la palipéridone et/ou ses sels de qualité pharmaceutique sous n'importe laquelle de leurs combinaisons, un copolymère biocompatible à base d'acide lactique et glycolique, ayant une proportion de monomère d'acide lactique à glycolique de l'ordre de 50:50 et du DMSO comme solvant, où la composition libère le médicament avec un début d'action immédiat et de façon continue pendant au moins 8 semaines et où la composition possède un profil pharmacocinétique *in vivo* adapté pour la formulation à administrer toutes les 8 semaines ou plus longues périodes, **caractérisée par le fait que** le copolymère biocompatible a un poids moléculaire entre 31 et 40 kDa et une viscosité inhérente de 0.27-0.31 dl/g.

**2.** La composition de la revendication 1, où le copolymère biocompatible est un gamma ou bêta irradié dans la gamme de doses de 10 à 30 KGy mesurées à une température comprise entre -40 °C et +35 °C pour adapter son poids moléculaire entre 31 et 40 kDa et sa viscosité inhérente entre 0.27-0.31 dl/g.

3. La composition des revendications 1 ou 2, où la distribution de la taille de la particule du médicament est :

   - moins de 10 % de particules inférieures à 10 microns ;
   - moins de 10 % de particules supérieures à 225 microns ; et
   - une valeur d0.5 dans la gamme de 40-130 microns.

4. La composition selon n'importe laquelle des revendications 1 à 3, où le rapport de masse médicament/(polymère + médicament) est d'environ 33 %.

5. La composition selon n'importe laquelle des revendications 1 à 4, où la teneur en médicament est environ de 13 % p/p du total de la formulation et la viscosité de la solution comprenant le polymère et le DMSO est entre 1.7-1.8 P.a.s.

6. La composition de n'importe laquelle des revendications précédentes pour le traitement de la schizophrénie ou des troubles bipolaires dans le corps humain.

7. Un kit pharmaceutique adapté pour la formation *in situ* de l'implant biodégradable dans un corps comprenant la composition de n'importe laquelle des revendications 1-6, où le médicament et le polymère biocompatible sont contenus dans un premier conteneur, et le solvant est contenu dans un second conteneur séparé.

8. Le kit pharmaceutique selon la revendication 7, où au moins l'un des premier et second conteneurs est une seringue, un flacon, un dispositif ou une cartouche, jetable ou pas.

9. Un procédé pour la fabrication d'une composition selon n'importe laquelle des revendications précédentes 1-6, comprenant l'étape consistant à fournir un copolymère biocompatible ayant un poids de polymère initial supérieur à celui qui est nécessaire pour la composition à effet retard intramusculaire et ensuite à adapter son poids moléculaire entre 31 et 40 kDa et sa viscosité inhérente entre 0.27-0.31 dl/g en l'irradiant par rayonnement gamma ou bêta dans la gamme de doses de 10 à 30 KGy mesurées à une température comprise entre - 40 °C et +35 °C.

10. Procédé selon la revendication 9 où la gamme de doses irradiées au polymère est entre 16-25 KGy mesurées à une température de 8 °C.

11. Procédé selon les revendications 9 ou 10 où, lorsque le polymère biocompatible possède un poids moléculaire initial d'environ 50 kDa, il est irradié avec une dose de radiation d'environ 16 KGy pour réduire son poids moléculaire entre 35 et 38 kDa.

12. procédé selon les revendications 9 ou 10 où, lorsque le polymère biocompatible possède un poids moléculaire initial d'environ 63 kDa, il est irradié avec une dose de radiation d'environ 30 KGy pour réduire son poids moléculaire entre 30 et 36 kDa.

**FIGURES**

**Figure 1**

**Figure 2**

EP 2 529 757 B1

Figure 3

Figure 4

Figure 5

17

**Figure 6**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 200617537 A
- WO 2008128436 A
- WO 200915828 A
- EP 2234617 A

- US 2009163519 A
- WO 201142453 A
- WO 2008153611 A2
- WO 2011151355 A

**Non-patent literature cited in the description**

- Basic definitions of terms relating to polymers. *Pure Appl. Chem.,* 1974, vol. 40, 477-491